# EUROPEAN PATENT APPLICATION

(11) **EP 4 349 370 A1**
(43) Date of publication of application: **10.04.2024**
(21) Application number: 21942854.7
(22) Date of filing: 28.05.2021
(51) Int. Cl.: A61K 47/10, A61K 31/352, A61K 39/395, A61K 9/107, A61P 31/12, A61P 19/02, A61P 17/00

(54) **TRANSCUTOL-POLYOL-ACTIVE AGENT TERNARY COMPLEX, PHARMACEUTICAL COMPOSITION FOR TOPICAL USE COMPRISING SAME, METHOD FOR OBTAINING SAME AND USE THEREOF IN DIFFERENT PATOLOGIES**

(71) Applicant: Fundación Gaiker, 48170 Zamudio (Bizkaia) (ES)
(72) Inventor: GOÑI DE CERIO, Felipe, 48170 ZAMUDIO (Bizkaia) (ES); PÉREZ LEBEÑA, Eduardo, 47014 VALLADOLID (ES)
(74) Representative: Ungria López, Javier
(86) International application number: PCT/ES2021/070385
(87) International publication number: WO 2022/248744

(57) **Abstract**

The present invention relates to a ternary complex that, owing to its formulation, improves the bioavailability of certain molecules inside the body which act as active agents and which, due to their structure, are not absorbed because their solubility in water is highly limited or because their ingestion via a route such as the gastric route is not advisable based on the degradation it causes in the molecule. Likewise, the present invention relates to a method for obtaining this complex, as well as a pharmaceutical composition comprising same, which is effective for preventing viral infectious processes, in addition to treating and preventing chronic and degenerative diseases by means of the antioxidant, anti-inflammatory and regenerative capacity of one of its components, the polyphenolic compounds.

## Description

### Technical field of the invention

The invention falls within the field of Organic Chemistry and is mainly applicable in the healthcare and pharmaceutical sector. It specifically relates to a composition intended to achieve, owing to its components, the solubilization and bioavailability of certain bioactive molecules inside the body which are not soluble in water or their ingestion via the gastric route is not advisable based on the associated molecular degradation. This complex, and the pharmaceutical formulation comprising same, are intended for topical use, being effective for preventing viral infectious processes, in addition to treating and preventing chronic and degenerative diseases by means of the antioxidant, anti-inflammatory and regenerative capacity of one of its components, which are polyphenolic compounds.

### Background of the invention

There is growing scientific interest in matters such as the need to develop new routes that are effective for systemically administering bioactive compounds. Among the routes that are already known, there exists the new route of skin penetration, hardly used until now, but which may be suitable and realistic for several reasons.

In this regard, improving permeability and dermal penetration emerges as a novel and feasible avenue of research to solve several problems:
- poor absorption of active agents, as occurs in many cases with oral absorption,
- impossibility or difficulty of absorbing compounds that are insoluble in water,
- appearance of side effects or the risks of frequently dosing pharmaceutical ingredients with negative consequences for the gastric route, for which their consumption requires the prescription of protectors such as omeprazole,
- degradation of certain compounds through the organism on their way to the part where they must carry out their effect, sometimes degrading irreversibly, as in the case of glycoproteins through the gastric route.

As an alternative route of administration, the skin provides an interesting opportunity to mitigate the aforementioned problems. However, the possibility of administering bioactive compounds via the dermal route is neither simple nor obvious, since it is affected and compromised by the formidable barrier properties of the skin itself. Ideally, an enhancer is usually selected for the composition, alone or in combination with other components, which reduces this barrier function of the skin, allowing the accompanying products to penetrate the skin.

In this sense, various studies have proven that transcutol, with IUPAC name 2-(2-Ethoxyethoxy) ethanol, CAS number: 111-90-0, is a compound with a potentiating effect on drug solubilization, as well as on the rate of percutaneous absorption and/or retention of drugs in the skin. In the United States, transcutol has been formulated in concentrations up to 49.9% in FDA-approved products for topical use. This excipient offers advantages over other enhancers because it is clear (transparent), non-volatile and nearly odorless, which favors its topical use.

Unlike other compounds of similar use such as oleic acid, azones and dimethyl sulfoxide (DMSO), transcutol does not compromise the integrity of the skin's structures. Given its superior solubilising power, unique physicochemical properties and a well-established safety profile, it is an ideal skin penetration enhancer. Based on available safety data, and considering a safety index of 100 times, the manufacturer recommends a limit of 20 mg/kg via the dermal route and 10 mg/kg via the oral route. Its physical and chemical properties are comparable to protic solvents such as ethanol (EtOH), propylene glycol (PG) and water. The viscosity, density and Log P properties of transcutol are close to those of water, but the vapor pressure and boiling point are close to those of PG. Other properties, such as the surface tension and melting point of transcutol, are between those of EtOH and water. The relatively low vapor pressure and high boiling point of transcutol are advantages over EtOH and water that cannot be processed at high temperatures. Transcutol is chemically stable at neutral and alkaline pH, with an optimal formulation range of pH 4 to 9. The pH of a transcutol solution up to 10% is almost neutral.

One important property of transcutol is its ability to dissolve a wide range of hydrophilic and lipophilic active agents. Its ability to surpass PG and EtOH in solubilizing power makes it a very useful pharmaceutical excipient. With a negative log P of ~0.5, it is considered a polar protic solubilizer that demonstrates affinity and good miscibility with hydrophobic groups.

Transcutol is compatible with most pharmaceutical excipients; it is soluble in common solvents such as glycerin, EtOH, propylene glycol (PG) and water, and is miscible with polar lipids such as medium-chain triglycerides and polyethylene glycol (PEG)-based surfactants (polyoxylglycerides).

However, despite its favorable properties, its use still poses problems. For example, skin permeation of transcutol is usually achieved by dilution with water, which can be explained by its high affinity for same. On the contrary, in the absence of this medium, pure transcutol is not attracted to the aqueous regions of the bilayers in the skin region, immobilizing and being retained on the skin's surface and, in some cases, the rigidity of the skin increases and its permeability decreases.

Moreover, polyphenols are a group of chemical substances found in plants, said substances being characterized by the presence of more than one phenol group in the aromatic part of the molecule. They are abundant in plants, and in foods, the main sources are berries, tea, beer, grapes and their derivative products (wine), olive oil, chocolate/cocoa, citrus peel and juice, walnuts, pomegranates, yerba mate, and other fruits and vegetables. More specifically, polyphenols account for up to 0.2-0.3% of fresh weight in many fruits and berries. Consuming common servings of wine, chocolate, legumes or tea can also contribute to the ingestion of approximately one gram per day. The most important food sources are basic products that are consumed in large quantities, such as fruits and vegetables, green tea, black tea, red wine, coffee, chocolate, olives and extra virgin olive oil. Herbs and spices, nuts and algae are also potentially important for supplying certain polyphenols. Some polyphenols are specific to certain foods (citrus flavanones, soy isoflavones, apple phloridzin), while others, such as quercetin, are found in all plant products, such as fruits, vegetables, grains, legumes, tea and wine. They contribute to bitterness, astringency, color, flavor, odor and oxidative stability in foods.

Polyphenols are structurally diverse. The term "polyphenol" is not clearly defined in the literature, but it is generally accepted that they are natural products containing several hydroxyl groups in aromatic rings in their structure, including four main classes based on the number of phenolic rings they contain and the structural elements that bind these rings together: phenolic acids, flavonoids, stilbenes and lignans:
- Flavonoids include flavones, flavonols, flavanols, flavanones, isoflavones, proanthocyanidins and anthocyanins. The most abundant flavonoids in foods are catechin (tea, fruits), hesperidin (citrus fruits), cyanidin (red fruits and berries), daidzein (soybeans), proanthocyanidins (apple, grape, cocoa) and quercetin (onion, tea, apple);
- Phenolic acid includes cinnamic acid, gallic acid, ferulic acid and caffeic acid;
- Lignans are phenylalanine-derived polyphenols found in flax seeds and other grains.
- Stilbene is a polyphenol having formula C₁₄H₁₂, the best-known representative of which is resveratrol, and it is found in many higher plants. Stilbenes make it possible to understand why some grapevine varieties are more or less resistant to fungal attacks.

The phenolic unit may be present in esterified, methylated, glycosylated, dimerized or polymerized form, creating a new class of polyphenol in all of them.

Polyphenols are usually macromolecules. Their molecular weight upper limit is about 800 daltons. Many larger polyphenols are biosynthesized in situ from smaller ones until becoming non-hydrolysable tannins and they remain undiscovered in the plant matrix. Most of them contain repeating phenolic molecules of pyrocatechol, resorcinol, pyrogallol and chloroglucinol connected by esters (hydrolysable tannins) or more stable C-C bonds (non-hydrolysable condensed tannins). Proanthocyanidins are mainly polymeric units of catechin and epicatechin.

Next, chemical structures of the different identified classes of polyphenols are shown.

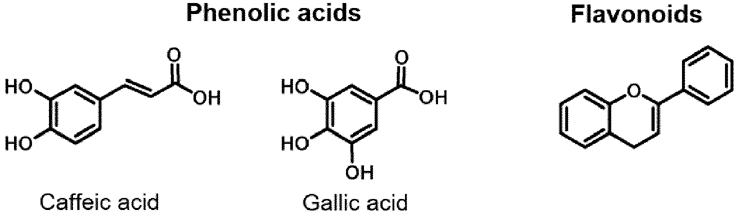

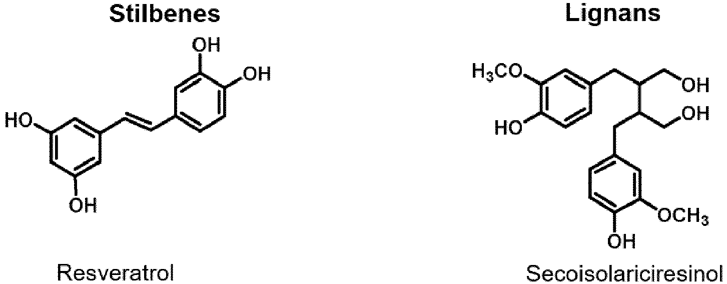

Phenolic acids are in turn divided into benzoic hydroxyl acids and cinnamic hydroxyl acids, and represent approximately one-third of the polyphenolic compounds in our plant diet, especially abundant in fruits with an acidic flavor. Flavonoids are the most abundant polyphenols in the human diet and share a common basic structure having two aromatic rings, bound by three carbon atoms that form an oxygenated heterocycle. One of the rings usually comes from a resorcinol molecule and the other comes from the shikimate pathway. Stilbenes contain two phenyl molecules connected by a two-carbon methylene bridge. Most stilbenes in plants act as antifungal phytoalexins, compounds that are synthesized only in response to infection or injury. The most studied stilbene is resveratrol. In turn, lignans are diphenolic compounds having a 2,3-dibenzylbutane structure formed by the dimerization of two cinnamic acid residues.

Polyphenols usually have functional groups beyond the hydroxyl groups, and the esterified bonds are shared, just like the carboxylic acids or the glycosylated groups. They are species reactive to oxidation, hence their definition as *in vitro* and *in vivo* antioxidants. Tannins were traditionally used to tan leather and today they are also precursors in green chemistry, especially for producing plastics or resins by polymerization with or without the use of formaldehyde or adhesives for particle boards.

At the end of the 20th century, epidemiological studies and the associated meta-analyses suggested that the long-term consumption of diets rich in plant polyphenols offered protection against the development of cancers, cardiovascular diseases, diabetes, osteoporosis and neurodegenerative diseases. Polyphenols and other dietary phenols are the subject of growing scientific interest due to their potential beneficial effects on human health. Flavonoids constitute the most studied group. They retain a common basic structure consisting of two aromatic rings bound by three carbon atoms that form an oxygenated heterocycle. More than 4,000 varieties have been identified, many of which are responsible for the attractive colors of flowers, fruits and leaves. Based on the variation in the type of heterocycle involved, flavonoids can be divided into six subclasses: flavonols, flavones, flavanones, flavanols, anthocyanins and isoflavones. Individual differences within each group arise from the variation in the number and arrangement of the hydroxyl groups and their degree of alkylation and/or glycosylation. Quercetin, myricetin, catechins, etc., are some of the most common flavonoids.

Chemical structures of the flavonoid subclasses are shown below.

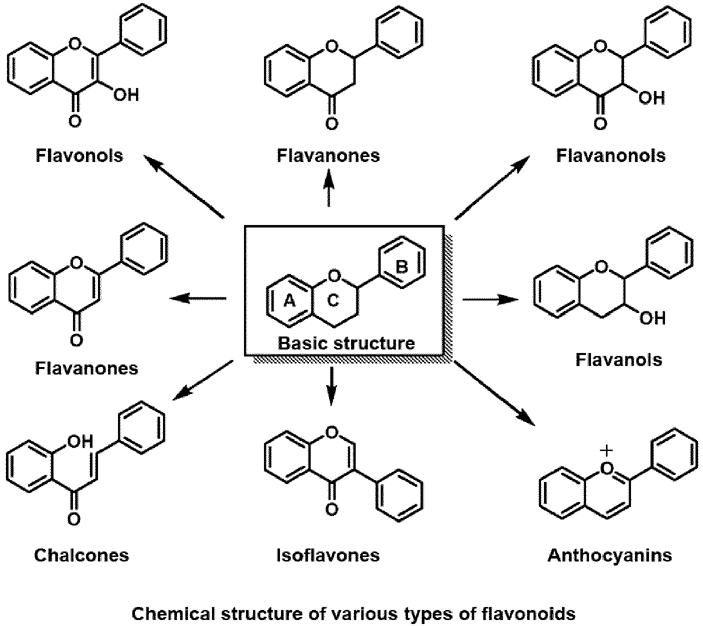

Individual differences within each group arise from the variation in the number and arrangement of the hydroxyl groups and their degree of alkylation and/or glycosylation. Flavonols (such as quercetin and kaempferol) have a 3-hydroxy-pyran-4-one group in the ring C. Flavanones (such as naringenin and taxifolin) have an unsaturated carbon-carbon bond in the ring C. Flavanols (such as catechins) lack both a 3-hydroxyl group and the 4-one structure in the ring C. Flavones (such as luteolin) lack a hydroxyl group at position 3 of the ring C. Anthocyanins (such as cyanidin) are characterized by the presence of an oxonium ion in the ring C and, as a result, they are very colorful, and in isoflavones (such as genistein), the ring B is bound to the ring C at position 3, instead of at position 2, as occurs with the other flavonoids.

Epidemiological studies have repeatedly demonstrated an inverse association between the risk of chronic human diseases and the consumption of a diet rich in polyphenols. Its phenolic groups can accept an electron to form relatively stable phenoxyl radicals, thus interrupting the chain reactions of oxidation in cellular components.

Foods and beverages rich in polyphenols increase the antioxidant capacity of plasma after consumption, associated with reduced levels of oxidative damage to cellular DNA. Similar observations have been made with foods and beverages rich in polyphenols, indicating the protective effects of polyphenols. As antioxidants, they protect cell components against oxidative damage and, therefore, limit the risk of various degenerative diseases associated with oxidative stress.

Dietary polyphenols retain a number of characteristics that make them unique, since they have pleiotropic effects on health. They are good antioxidants and can neutralize the destructive reactivity of unwanted reactive oxygen/nitrogen species, which arise as a byproduct during metabolic processes in the body. Polyphenols provide significant protection against the development of several chronic diseases such as cardiovascular diseases (CVD), cancer, diabetes, infections, ageing, asthma, etc. Specifically, flavonoids are associated with very diverse positive effects on health, as anti-viral, anti-diabetic, anti-gout, anti-bacterial, anti-thrombotic, anti-carcinogenic, anti-hypertensive, anti-inflammatory, cardioprotective and antioxidant agents.

There is an association between the ingestion of foods rich in polyphenols and the incidence of cardiovascular diseases or cancer-related diseases: on the one hand, it has been observed that the consumption of a diet rich in polyphenols is associated with a lower risk of myocardial infarction in both case-control and cohort studies. Thus, it has been observed that resveratrol, the polyphenol in wine, prevents platelet aggregation and is capable of relaxing arteries and aortic rings. On the other, theaflavins and catechins, abundant in black and green tea, have been shown to exhibit anti-cancer properties, since they inhibit proliferation and increase apoptosis in prostate carcinoma cells; the addition of black tea polyphenols at a dose of 40 mg/ml blocks metastatic cell progression in prostate carcinoma cells.

Furthermore, the combination of antioxidant/anti-inflammatory polyphenolic compounds from fruits and vegetables are effective as anti-ageing compounds. Anthocyanins, abundant in brightly colored fruits, such as berries and grapes, are responsible for same, and they exhibit powerful antioxidant/anti-inflammatory activity and inhibit lipid peroxidation and the inflammatory mediators of cyclooxygenase (COX)-1 and -2. In turn, spinach, strawberries and blueberries exhibit high total antioxidant activity. A recent study shows that tea catechins have strong anti-aging activity and their consumption can delay its onset.

Although health effects can be attributed to the polyphenols present in foods, the extensive metabolism of polyphenols in the intestine and liver, and their fate as metabolites that are excreted in urine, make it impossible to define their biological effects. Since the metabolism of polyphenols cannot be evaluated *in vivo,* no dietary reference ingestion levels have been established or recommended. For this reason, *in vivo* polyphenol supplementation studies remain inconclusive due to:
1) non-dietary polyphenols used in cosmetics (resveratrol, ferulic acid, etc.) are usually insoluble in water and their internal absorption is very limited for this reason, which represents a very important unfavorable condition in their use; and
2) the applications of the studies carried out *in vitro* are subsequently irrelevant to the design of *in vivo* experiments, since solvents are used that cannot then be extrapolated due to their health implications (dimethyl sulfoxide (DMSO), etc.).

Most polyphenols are present in foods in the form of esters, glycosides or polymers. Before they are absorbed, these compounds are hydrolyzed by intestinal enzymes or by colonic microflora. During absorption, the polyphenols undergo extensive modification; in fact, they are initially conjugated in the intestinal cells and then in the liver, through methylation, sulphation and/or glucuronidation reactions. Consequently, the forms that reach the blood and tissues are different from those present in foods and it is very difficult to identify all the metabolites and evaluate their biological activity. It is important to note that the chemical structure of polyphenols, and not their concentration, is what determines the rate and degree of absorption and the nature of the metabolites circulating in the plasma. The most common polyphenols in our diet are not necessarily those that have a higher concentration of active metabolites in target tissues. Indirect proof of their absorption through the intestinal barrier is given by the increase in the antioxidant capacity of plasma after consumption of foods rich in polyphenols.

Polyphenols also differ in their site of absorption in humans. Some are absorbed in the small intestine, while others are absorbed in the colon. In foods, all flavonoids, except flavanols, exist in glycosylated forms. Most glycosides resist stomach acid hydrolysis and, therefore, reach the intestine intact, where aglycones and glycosides are absorbed separately. It has been shown that, in rats and mice, anthocyanins are absorbed in the stomach.

Hydroxycinnamic acids, when ingested freely, are rapidly absorbed by the small intestine and are conjugated like flavonoids. However, in plant products, these compounds are naturally esterified and this makes their absorption difficult, since the intestinal mucosa, the liver and the plasma do not have esterases capable of hydrolyzing chlorogenic acid to release caffeic acid, and hydrolysis can only be carried out by the microflora present in the colon.

The aglycones are split by the opening of the heterocycle at different points, depending on its chemical structure, and thus produce different acids that are subsequently metabolized into benzoic acid derivatives. After absorption, the polyphenols go through several metabolic processes, which mainly include methylation, sulphation and glucuronidation, representing a metabolic detoxification process, common to many xenobiotics, which facilitates their biliary and urinary elimination by increasing their hydrophilicity. Methylation is also quite specific; it usually occurs at position C3, but it could occur at position C4'; in fact, a notable amount of 4'-methylepigallocatechin has been detected in human plasma after ingestion of tea.

Sulphation occurs in the liver, while glucuronidation occurs in the intestine and liver, the highest conjugation rate being observed at position C3. Conjugation mechanisms are efficient and free aglycones are usually absent or in low concentrations in plasma after consumption of nutritional doses. One exception is the catechins in green tea, the aglycones of which may constitute a significant proportion of the total amount in plasma.

Polyphenol metabolites that are bound to proteins, in particular, with albumin, circulate in the blood, playing an important role in their bioavailability. The affinity for albumin varies depending on its chemical structure and this affects the elimination rate of metabolites and their presence in cells and tissues.

The accumulation of polyphenols in tissues is the final phase of their metabolism, since it could reach a biologically active concentration capable of producing beneficial effects. Studies indicate that they are capable of penetrating tissues, especially the intestine and liver. Polyphenols with their derivatives are excreted through urine and bile. It has been observed that conjugated metabolites are more likely to be eliminated in the bile, while the small conjugates, such as monosulfates, are preferably excreted in the urine. The percentage of urinary excretion is quite high in the case of citrus fruit flavanones and decreases from isoflavones to flavonols. Thus, the beneficial health effects of polyphenols depend on both ingestion and bioavailability. Bioavailability is the proportion of the nutrient that is digested, absorbed and metabolized through normal pathways, and it is different for each polyphenol. In general, it can be stated that the bioavailability of polyphenols is naturally low or zero, due to their properties.

Moreover, the compounds that are usually used as active agents with pharmacological effects include antibodies, which are glycoproteins that belong to the immunoglobulin superfamily. The terms "antibody" and "immunoglobulin" are often used interchangeably, although "antibody" is sometimes reserved for the soluble and secreted form.

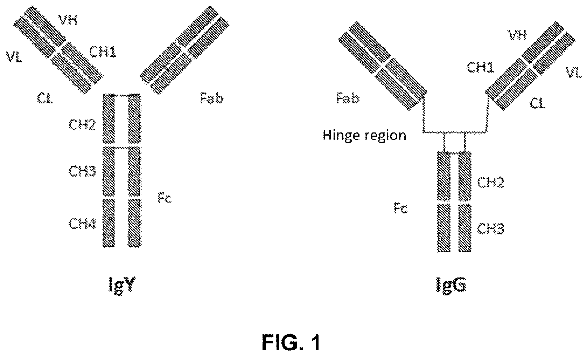

An Ab antibody is a large, Y-shaped protein (see Figure 1) that the immune system uses to identify and neutralize foreign objects, such as pathogenic bacteria and viruses. The antibody recognizes a unique molecule of the pathogen, called antigen. Each tip of the "Y" contains a specific paratope (analogous to a lock) for a specific epitope (analogous to a key) of an antigen, allowing these two structures to be bound together precisely. Due to this mechanism, an antibody marks a microbe or an infected cell to be attacked by other parts of the immune system, or to be neutralized by said parts (for example, blocking a part of a virus that is essential for its invasion).

In order for the immune system to recognize millions of different antigens, the antigen-binding sites located at the two ends of the antibody exhibit an equally wide variety. In contrast, the rest of the antibody is relatively constant. It is only exhibited in a few variants, which define the class or isotype of the antibody: IgA, IgD, IgE, IgG or IgM. The constant region on the antibody stem includes sites that participate in interactions with other components of the immune system. The class, therefore, determines the function that an antibody triggers after binding to an antigen, in addition to some structural characteristics. Antibodies of different classes also differ in the area of the body in which they are released and in the phase of the immune response.

Along with B and T cells, antibodies are the most important part of the adaptive immune system. Soluble antibodies are released into the blood and tissue fluids, as well as in many secretions. Since these fluids were traditionally known as humoral fluids, antibody-mediated immunity is known as humoral immunity.

In the prenatal and neonatal stages, the presence of antibodies is determined by the passive immunization of the mother. Antibodies contribute to immunity in three ways: i) they prevent pathogens from entering or damaging cells by binding to them, ii) they stimulate the elimination of pathogens by macrophages and other cells by coating the pathogen, and iii) they trigger the destruction of pathogens by stimulating other immune responses such as the complement pathway.

A typical IgG1 antibody contains two identical light chains and two identical heavy chains. It contains a total of 16 disulphide bonds, including 4 interchain bonds in the hinge region and 12 intrachain bonds associated with 12 individual domains. The disulphide bond structure is fundamental to the structure, stability and biological functions of IgG molecules. Interchain disulphide bonds are known to be more susceptible to reduction than intrachain disulphide bonds.

Disulphide bonds are a covalent bond between two sulphur atoms. This bond is preferably weak, with a typical bond dissociation energy of 60 kcal/mol; therefore, in the presence of a strong acid such as hydrochloric acid, along with reducing conditions in the medium, the disulphide bond is broken forming a thiol (-SH), this being a more stable bond.

In the relevant case of the current COVID-19 pandemic, responses against the SARS-CoV-2 spike protein have been characterized and correlate with the titers of IgG and IgA antibodies present in patients. CD8+ T cells are cytotoxic and play a critical role in eliminating the virus. The total lymphocytes, CD4+ T cells, CD8+ T cells, B cells and natural killer NK cells showed a significant association with the inflammatory state in COVID-19, especially with CD8+ T lymphocytes and the CD4+/CD8+ ratio. IFN-γ expression by CD4+ T cells also tends to be lower in severe cases compared to moderate cases. Detecting antibodies against SARS-CoV-2 does not directly indicate protective immunity, and correlates of protection for COVID-19 have not yet been established. Most infected people exhibit a humoral response between days 10 and 21 after infection. Detection in mild cases may take longer, four weeks or more, and in a small number of cases the antibodies (IgM, IgG) are not detected. According to available data, IgM and IgG antibodies against SARS-CoV-2 develop between 6 and 15 days after the onset of the disease. The median seroconversion time for total antibodies IgM and then IgG was on day 11, day 12 and day 14 after the onset of symptoms, respectively. The presence of antibodies was detected in <40% of patients in the first week after onset, and rapidly increased to 100% (total antibodies), 94.3% (IgM) and 79.8% (IgG) as of day 15. Longevity of the antibody response is still unknown, but antibodies against other coronaviruses are known to decrease over time (12-52 weeks after the onset of symptoms) and homologous reinfections have been confirmed. IgM and IgG antibody levels against SARS-CoV-2 can remain for seven weeks or at least in 80% of cases until day 49. In comparison, 90% and 50% of patients infected with SARS-CoV-1 maintain IgG antibodies for two and three years, respectively.

Various studies have been carried out to try to use mammalian antibodies in enriched serum, but this solution has been discarded. In contrast, it has been observed that, by immunizing hens, said hens generate IgY antibodies that can be homologous to humans. Specifically, and this makes the system industrially scalable, these antibodies are transmitted and deposited in the yolks of eggs laid by the immunized hens (their offspring). Therefore, the egg yolk constitutes an important alternative source of antibodies. It exhibits some advantages over mammalian serum immunoglobulins in terms of productivity, animal welfare and specificity. The main immunoglobulin present in avian blood (IgY) is transmitted to their offspring and accumulates in the egg yolks, which enables the non-invasive obtaining of high amounts of antibodies. Furthermore, due to structural differences and phylogenetic distance, IgY is more suitable for diagnostic purposes than mammalian antibodies, since it does not react with certain components of the human immune system and shows greater avidity for mammalian conserved proteins. The specific protective effect of egg yolk extracts from immunized hens, attributed to the transfer of serum hen antibodies to eggs, has created new interest due to the pursuit of animal welfare. Thus, in 1996, the European Centre for the Validation of Alternative Methods (ECVAM) recommended the use of IgY rather than mammalian IgG, with the purpose of minimizing the pain caused by the invasive collection of serum antibodies. IgY is present in birds, reptiles, amphibians and lungfish and is the evolutionary precursor of IgG and IgE, present only in mammals. The IgY molecule has a structure similar to that of IgG, with two heavy chains (H), each with a molecular weight of 67 to 70 kDa, and two light chains (L), of 25 kDa. The main difference between IgY and IgG is found in the heavy chains. IgG has three constant regions in the heavy chains (CH1-CH3), while IgY has four constant regions (CH1-CH4).

IgY is more hydrophobic than IgG and has an isoelectric point between 5.7 and 7.6. The half-life of purified IgY lasts months, retaining activity for up to six months at room temperature and one month at 37°C. Even though it is protein, IgY is resistant to heat and pH, being stable between 30° and 70°C and active between pH 3.5 and 11. However, the affinity of IgY to its antigen decreases with increasing temperature. IgY is resistant to inactivation by the proteolytic enzymes trypsin and chymotrypsin, but it is degraded by pepsin, a digestive enzyme present in the stomach of mammals where it hydrolyses proteins. Pepsin acts as a protease and degrades Ig, this being the problem of the gastric route for these antibodies. The digestion of IgY with papain leads to a Fc fragment plus two monovalent Fab fragments, such as IgG.

Indeed, the greatest interest in the search for new routes of drug administration, such as the transdermal route, arises from the need to complement and improve existing therapies and explore alternative routes of administration, in particular for active pharmaceutical ingredients with undesirable oral absorption characteristics, mainly due to the following pharmacopoeia problems:
- the lack of solubility in water of certain molecular structures, such as polyphenols; and
- avoiding ingestion of glycoproteins via the gastric route, which is not advisable based on the molecular denaturation and degradation that is generated by the acidity of the stomach and the presence of proteases such as pepsin.

The present invention has been developed in view of the foregoing and the problems detected in the technical field, which essentially relates to the low bioavailability of certain active agents, which leads to low assimilation of same by the organism, and the degradation that these or other agents undergo in the usual routes of drug administration. This invention focuses on obtaining a ternary complex formed by the active agent and components that, acting as a topical carrier (and also with adverse and beneficial effects for the organism), promote bioavailability and prevent degradation during administration.

### Description of the invention

The present invention is based on the results found through experiments in the search for new formulas to implement the use of active agents in the field of medicine, on the one hand to promote their absorption in the organism, such as polyphenols, and, on the other hand, to avoid gastric degradation, such as glycoproteins (like the IgG or IgY antibodies), in an attempt to ensure that these compounds reach their organic targets in an optimized way and can fulfil their function. Otherwise, their bioavailability and absorption in the body are compromised, as mentioned in the previous section.

Thus, the object of the present invention is a ternary complex that contains said active agents, the pharmaceutical composition for topical use comprising same, such as a topical solution in the form of a cream or a patch, and the use of same in the field of medicine.

Specifically, in a first aspect, the invention relates to a ternary complex formed by:
- transcutol, which is diethylene glycol monoethyl ether (2-(2-Ethoxyethoxy)ethanol, having formula CH₃CH₂OCH₂CH₂OCH₂CH₂OH),
- a solvent, which is a polyol, and
- an active agent, which is selected from a) one or more polyphenols or b) an antibody;
   wherein the ratio by weight of transcutol:polyol:active agent is between:
      ∘ 1:4.8:1 and 1:4.8:1.6 when the active agent is at least one polyol;
      ∘ 1:4.8:0.075 and 1:4.8:0.125 when the active agent is an antibody;
   and wherein the active agent is bound to the transcutol and to the polyol through weak bonds by hydrogen bridges.

With regards to the ratio by weight of the three components, transcutol:polyol:active agent, said ratio can be, for example, between 25 g:120 g:25 g and 25 g:120 g:40 g when the active agent is one or more polyphenols, or between 25 g:120 g:3 g and 25 g:120 g:5 g when the active agent is an antibody.

The ternary complex is presented in the form of a liquid solution, which is a polyol.

Transcutol, which is commercially known, is configured as a safe enhancing agent for a wide range of active drugs, since it optimizes the absorption and penetration of same through the dermis and epidermis. In this invention, it has been observed that transcutol gives stability to the polyol:active agent complex and, on the other hand, it improves absorption and penetration through the dermis and epidermis. Furthermore, it has been found that the active agent dissolves in the polyol better than in transcutol alone, but once it is dissolved in the polyol, transcutol ends up further homogenizing the complex and giving it greater stability and penetrability.

The polyol is preferably selected from propylene glycol and glycerin, propylene glycol being the most preferred. The solubility of polyphenols in short chain alcohols, such as methanol or ethanol, is reduced, and their use is also invalidated when taking into account their toxicity. In contrast, due to the present invention it has been found that their solubility increases drastically when dissolved in polyols, such as propylene glycol and glycerol. In this case, as will be discussed later, the solubility of polyphenols is achieved at a temperature between 30° and 40°C, subsequently maintaining said solubility in water at room temperature (where "at room temperature" is understood as "around 25°C"), forming part of the complex, after being previously dissolved in the aforementioned polyols.

This greater solubility in water of the complex compared to the separate components represents a very relevant advantage in the field, since it is an important property for polyphenol activity. Since the product in which the ternary complex will be integrated as an active compound, which is a pharmaceutical composition (for example, a cream), will be manufactured at 40-50°C, it is important for the polyphenol to continue to maintain solubility in water when the composition is made and its homogeneity achieved. Owing to the inclusion of polyphenol in the complex, increased solubility is maintained in the emulsion when the pharmaceutical composition is prepared, something that had not been achieved until now when preparing pharmaceutical compositions with polyphenols.

The polyphenol, one of the two types of active agents that can be selected in this invention, has antioxidant and anti-inflammatory characteristics. This polyphenol can be any polyphenol that is commercially available on the market, although it is preferably a flavonoid, since it exhibits more pronounced beneficial properties (in this sense, it should be noted that resveratrol, which is a stilbene, also has those increased characteristics, which is due to the existence of a double polyphenolic group). Thus, the flavonoid can be selected from the group consisting of: apigenin, naringenin, rutin, hesperidin, diosmin, catechin, epicatechin, isorhamnetin, xeractinol, kaempferol, myricetin or quercetin, cyanidin, delphinidin, malvidin, peonidin, petunidin, silymarin (particularly, silybin), as well as any of its constituents separately and any combination thereof. Even more preferably, the flavonoid of the complex is rutin. In the most preferred case of all, the polyphenol is quercetin, which is the aglycone of rutin. In a particular case, the ternary complex comprises only one type of polyphenol, while in another particular case the ternary complex may contain different polyphenols, for example a mixture of rutin and resveratrol.

In turn, the antibody, which is the other active agent that can be selected, is preferably an immunoglobulin selected from the group consisting of IgA, IgD, IgE, IgG, IgM and IgY, where IgY is in the most preferred. In a highly preferred particular case, the IgY antibodies come from avian organisms (i.e., birds), of the same or different species, since avian IgY can be easily collected from hen eggs in large quantities, their production can be industrially scaled and their purification is simple and cost-effective. Preferably, the bird is a gallinaceous bird, more preferably of the species *Gallus gallus domestica.* In the most preferred case, the avian organism is a female. In the most preferred case of all, it is a hen.

Specifically, when the antibodies come from avian organisms, the antibodies are extracted from eggs of the aforementioned birds. This offers a very relevant advantage: antibodies are obtained by non-invasive methods, and the complications derived from using mammalian antibodies are also reduced.

Using these antibodies involves, essentially, immunizing avian organisms with antigens, so that they produce the corresponding antibodies that are found in egg yolks, and subsequently extracting these antibodies from said yolk. This process usually requires a subsequent step of purifying the different extracted IgY antibodies.

Likewise, a second object of the present invention relates to a method for obtaining the ternary complex described, in any of its variants disclosed herein. Said method comprises:
a) preparing a solution of the active agent, which can be selected from one or more polyphenols or an antibody, in a solvent that is polyol (preferably, propylene glycol), by vigorously stirring a mixture (understood to be for at least 500 rpm for a time between 15 and 25 minutes, even more preferably for at least 20 minutes) and at a temperature above room temperature (i.e., higher than 25°C) and lower than 40°C, preferably between 30° and 40°C, including both limits; and
b) adding the transcutol to the previous solution, and subjecting to cavitation induced by 20 kHz ultrasound, for a time of at least 5 minutes until complete mixture of the components is achieved, where the complexes are formed in a solution through weak intermolecular interactions between the hydroxyl groups present in the three components.

When polyphenol is used as an active agent, ternary complexes are formed in a solution through weak intermolecular interactions due to bonds by hydrogen bridges between the hydroxyl groups of the polyphenol and the hydroxyl groups of the polyol. In the case of the antibody as an active agent, the complexes are formed by forming weak bonds by hydrogen bridges with the other two components. For example, propylene glycol, the preferred polyol, has two OH groups, just like the 2 OH groups of polyphenol. In turn, the antibody, which is a glycoprotein, has OH groups of the gluco and amino groups of the protein, which also form bonds by hydrogen bridges. Transcutol has a terminal OH group. All of these hydroxyl groups facilitate the dissolution of the components, at the same time that they form weak forces that retain said components.

In the particular case where the active agent is an antibody, said antibody can be found at the origin before step a) in one of the following forms: in an aqueous solution, which is how it is obtained from egg yolks, or lyophilized, i.e., in solid state, which is how it should be normally added to the mixture in step a). For this reason, the method described for obtaining the ternary complex may include a previous step that consists of lyophilizing an aqueous solution of the antibody acting as an active agent in the final complex.

The antibodies, preferably when they are IgY, can be previously obtained and purified by bird immunization methods, as described in international patent applications WO 2007124755 and WO 2007079755.

Synthesizing the complex by ultrasonic cavitation is similar to the methods described in Spanish patents P201731489 and P202030007, where ultrasonic cavitation is used, prior to heating the components, or in Spanish patent application P202030273, where dispersion by stirring is described, prior to heating the components, and that have been carried out by inventors of the present invention.

A third object of the present invention is a pharmaceutical composition comprising a ternary complex such as the one described previously as an active compound. Preferably, the complex, understood as an active ingredient, is comprised in the pharmaceutical composition in a ratio by weight with respect to the total weight of the composition selected from:
∘ 1:4.8:1 and 1:4.8:1.6 for each kilogram of pharmaceutical composition, when the active agent is at least one polyphenol; and
∘ between 1:4.8:0.075 and 1:4.8:0.125 for each kilogram of pharmaceutical composition, when the active agent is an antibody.

With regards to the ratio by weight of the three components, transcutol:polyol:active agent, said ratio can be, for example, between 25 g:120 g:25 g and 25 g:120 g:40 g for each kilogram of pharmaceutical composition when the active agent is one or more polyphenols, or between 25 g:120 g:3 g and 25 g:120 g:5 g for each kilogram of pharmaceutical composition when the active agent is an antibody.

In the most preferred case, the pharmaceutical composition is for topical use, and can be in a form selected from, for example, a topical solution or a patch. The topical solution, which is the most preferred of all the pharmaceutical compositions, can, in turn, be preferably selected from a cream or an emulsion. In this case, the composition may include excipients and adjuvants that are commonly used for preparing medicines of this class.

Thus, to prepare the pharmaceutical composition from the ternary complex, the usual methods of mixing the active compound, which is the complex, with the rest of the excipients that make up the topical solution, regardless of its form (emulsion or cream), are simply followed. In fact, it should be noted that the pharmaceutical composition for topical use, being a topical solution such as a cream, can be obtained in two alternative ways that achieve the same result: either by a) preparing the cream or emulsion to then subsequently add the ternary complex as an active compound of the composition, or b) preparing the cream or emulsion at the same time that the ternary complex is obtained, according to the foregoing specifications.

Another object of the present invention is a pharmaceutical composition for use in medicine, specifically for use thereof in treating and/or preventing a disease selected from the group consisting of: viral infectious processes, chronic and degenerative diseases, subcutaneous inflammations such as fasciitis and fasciosis, tendon inflammation and irritation (tendonitis), carpal tunnel inflammation, varicose vein reduction, joint inflammation, blows and bruises. This object thus includes the method for treating said diseases or processes by administering the pharmaceutical composition, including the use of a composition such as the one described in the manufacture of a medicine for the indicated treatments.

Preferably, the viral infectious processes are selected from the group consisting of DNA virus or RNA virus infections: HIV, SARS, MERS, Dengue, IAV or HCV, among others. Likewise, the diseases are preferably selected from the group of: subcutaneous inflammatory processes such as carpal tunnel inflammation, fasciitis, tendonitis, varicose veins, bruises and blows, arthritis, as well as chronic and inflammatory skin diseases such as psoriasis or atopic dermatitis.

Regarding the dosage for preventing and treating these diseases when the pharmaceutical composition is a topical solution, it is recommended that each application contains between 3 and 5 g of cream, such that it is applied daily 2 to 3 times for prevention, and 3 to 6 times for treatment.

### Brief description of the figures

Figure 1 represents the structure of IgY and IgG. V = light chain variable domain (VL) and heavy chain variable domain (VH); C = light chain constant domain (CL) and heavy chain constant domain (CH).

### Exemplary embodiments

### Example 1: Preparation of the ternary complex object of protection, with polyphenol as an active agent

40 g of rutin were weighed and 125 g of propylene glycol (PG) were weighed. PG was heated to 35°C and rutin was added. It was then stirred vigorously for 20 minutes at a speed of 500 rpm, until achieving initial solubilization of the flavonoid. Next, 25 g of transcutol were added, stirring intensely until the rutin is completely solubilized. After stirring the mixture, the obtained solution is subjected to sonication, with 20 kHz ultrasound for 5 minutes, to complete solubilization and finish producing the formation of bonds by hydrogen bridges.

Thus, the ternary complex was obtained in the form of a solution, suitable for the manufacture of pharmaceutical compositions, specifically for topical use, such as a cream.

### Example 2: Preparation of the ternary complex object of protection, with an antibody as an active agent

5 g of the IgY antibody were weighed, once purified and lyophilized, and 125 g of propylene glycol were weighed. A propylene glycol solution was heated to 35°C and IgY was added. Subsequently, it was stirred vigorously until the IgY antibody was solubilized and then 25 g of transcutol were added, stirring intensely until achieving total solubilization of the IgY.

After this phase, the obtained solution is subjected to sonication, to complete solubilisation and finish producing the formation of bonds by hydrogen bridges. Thus, this obtained solution can be used to manufacture creams or patches, for topical use.

### Example 3: Preparation of a pharmaceutical composition for topical use that is a cream based on the ternary complex of Example 1 and 2, which is the active compound

With regards to Example number 1, 810 g of the base cream are added to 190 g of the complex obtained in order to obtain 1 kg of total pharmaceutical composition.

With regards to Example number 2, 845 g of the base cream are added to 155 g of the complex obtained in order to obtain 1 kg of total pharmaceutical composition.

### Example 4: Application of the cream prepared in example 4 for treating diseases

In the numerous tests that were carried out on sick people, it has been observed that the introduction of a correct concentration of polyphenols (with "correct" being understood as the fact that there are 200 mg of the polyphenol in each 5 g application of pharmaceutical composition) in a solution for topical use (preferably a cream) has very beneficial effects in treating at least the following pathologies: subcutaneous inflammations such as fasciitis and fasciosis, tendon inflammation or irritation (tendonitis), carpal tunnel inflammation, varicose vein reduction, joint inflammation and blows and bruises.

In addition, and very surprisingly, it was observed that a 94-year-old patient, with significant varicose veins in the lower part of both legs, and after three months of daily treatment with a cream containing the bioflavonoid rutin (the aglycone of which is quercetin), partially recovered her cognitive abilities and once again recognised her family members, since she was experiencing a senile process of Alzheimer's disease. This indicates that, along with the ability of the mixture made of rutin, propylene glycol and transcutol to pass through the skin, the flavonoid in turn was able to cross the blood-brain barrier and produce its antioxidant and anti-inflammatory properties in the brain itself.

## Claims

1. A ternary complex formed by:
- diethylene glycol monoethyl ether, called transcutol,
- a solvent, which is a polyol, and
- an active agent, which is selected from a) one or more polyphenols or b) an antibody;
wherein the ratio by weight of transcutol:polyol:active agent is between:
∘ 1:4.8:1 and 1:4.8:1.6 when the active agent is at least one polyol;
∘ 1:4.8:0.075 and 1:4.8:0.125 when the active agent is an antibody;
and wherein the active agent is bound to the transcutol and to the polyol through weak bonds by hydrogen bridges.

2. The ternary complex according to claim 1, wherein the polyol is propylene glycol.

3. The ternary complex according to any one of claims 1 or 2, wherein the polyphenol is selected from a flavonoid or a stilbene.

4. The ternary complex according to claim 3, wherein the flavonoid is selected from the group consisting of: apigenin, naringenin, rutin, hesperidin, diosmin, catechin, epicatechin, isorhamnetin, xeractinol, kaempferol, myricetin or quercetin, cyanidin, delphinidin, malvidin, peonidin, petunidin, silymarin (particularly, silybin), as well as any of its constituents separately and any combination thereof; and the stilbene is resveratrol.

5. The ternary complex according to claim 4, wherein the flavonoid is rutin.

6. The ternary complex according to claim 5, wherein the flavonoid is the aglycone of rutin, called quercetin.

7. The ternary complex according to any one of claims 1 to 6, wherein the complex contains one or two different polyphenols.

8. The ternary complex according to any one of claims 1 to 7, wherein the antibody is an immunoglobulin selected from the group consisting of IgA, IgD, IgE, IgG, IgM and IgY.

9. The ternary complex according to claim 8, wherein the antibody is an IgY antibody from avian organisms of the same or different species.

10. The ternary complex according to claim 9, wherein the avian organism is a gallinaceous female.

11. The ternary complex according to any one of claims 9 or 10, wherein the antibody is obtained from the egg yolk of the avian organism.

12. A method for obtaining the ternary complex defined in any one of claims 1 to 11, **characterised in that** it comprises:
a) preparing a solution of the active agent in the polyol, which acts as a solvent, by vigorously stirring a mixture at, at least, 500 rpm and for a time between 15 and 25 minutes and at a temperature between room temperature and 40°C; and
b) adding the transcutol to the solution, and subjecting the solution to cavitation induced by 20 kHz ultrasound, for a time of at least 5 minutes until complete mixture of the components is achieved, where the complexes are formed in a solution through weak intermolecular interactions between the hydroxyl groups present in the three components.

13. The method according to the preceding claim, wherein the polyol is propylene glycol.

14. The method according to any one of claims 12 or 13, which comprises starting from an aqueous solution of the antibody, which is purified and lyophilized before step a).

15. A pharmaceutical composition comprising a ternary complex as defined in any one of claims 1 to 11, as an active compound.

16. The pharmaceutical composition according to the preceding claim, comprising the ternary complex in a ratio by weight with respect to the total weight of the composition of:
∘ 1:4.8:1 and 1:4.8:1.6 per kilogram of pharmaceutical composition, when the active agent is at least one polyphenol; and
∘ between 1:4.8:0.075 and 1:4.8:0.125 per kilogram of pharmaceutical composition, when the active agent is an antibody.

17. The pharmaceutical composition according to any one of claims 15 or 16, which is for topical use.

18. The pharmaceutical composition according to the preceding claim, which is a topical solution, selected from a cream or an emulsion.

19. A ternary complex defined in any one of claims 1 to 11 or a pharmaceutical composition comprising same defined according to any one of claims 15 to 18, for use thereof in medicine.

20. The ternary complex or the pharmaceutical composition comprising same for use according to the preceding claim, in treating or preventing a disease selected from the group consisting of: viral infectious processes, chronic and degenerative diseases, subcutaneous inflammations, tendon inflammation and irritation, carpal tunnel inflammation, varicose vein reduction, joint inflammation, blows and bruises.

21. The ternary complex or the pharmaceutical composition comprising same for use according to the preceding claim, wherein:
- the viral infectious process is selected from the group consisting of: DNA virus or RNA virus infections;
- the chronic and inflammatory skin disease is selected from psoriasis or atopic dermatitis;
- subcutaneous inflammation is selected between fasciitis or fasciosis; and
- joint inflammation is arthritis.

22. The ternary complex or the pharmaceutical composition comprising same for use according to any one of claims 19 to 21, which is presented in the form of a cream topical solution and is administered in an amount of 3 to 5 grams of cream, 2 to 3 times a day for prevention and 3 to 6 times a day for treatment.
